## Europäisches Patentamt
## European Patent Office
## Office européen des brevets

(19)

(11) Veröffentlichungsnummer : **0 005 467**
**B1**

(12)

# EUROPÄISCHE PATENTSCHRIFT

(45) Veröffentlichungstag der Patentschrift :
27.05.81

(51) Int. Cl.³ : **C 07 C 69/16**, C 07 C 67/05

(21) Anmeldenummer : 79101271.9

(22) Anmeldetag : 27.04.79

(54) Verfahren zur Herstellung von Diestern von olefinisch ungesättigten 1,2- und/oder-1,4-Diolen.

(30) Priorität : 11.05.78 DE 2820519

(43) Veröffentlichungstag der Anmeldung :
28.11.79 (Patentblatt 79/24)

(45) Bekanntmachung des Hinweises auf die Patenterteilung : 27.05.81 Patentblatt 81/21

(84) Benannte Vertragsstaaten :
**BE CH DE FR GB IT NL**

(56) Entgegenhaltungen :
**DE-A-2 012 903**
**GB-A-1 120 469**

(73) Patentinhaber : **BASF Aktiengesellschaft**
**Carl-Bosch-Strasse 38**
**D-6700 Ludwigshafen (DE)**

(72) Erfinder : **Weitz, Hans-Martin, Dr.**
**Auf dem Koeppel 40**
**D-6702 Bad Duerkheim 1 (DE)**
Erfinder : **Hartig, Juergen, Dr.**
**In der Schleit 9**
**D-6718 Gruenstadt (DE)**
Erfinder : **Marosi, Laszlo, Dr.**
**Londoner Ring 11**
**D-6700 Ludwigshafen (DE)**

Imprimerie Jouve, 17, rue du Louvre, 75001 Paris, France

EP 0 005 467 B1

# Verfahren zur Herstellung von Diestern von olefinisch ungesättigten 1,2- und/oder -1,4-Diolen

Die Erfindung betrifft ein Verfahren zur Herstellung von Diestern von olefinisch ungesättigten 1,2- und/oder 1,4-Diolen, insbesondere von Butendioldiacetaten, d.h. Buten-2-diol-1,4-diacetat und Buten-1-diol-3,4-diacetat, durch Umsetzung von konjugierten Diolefinen mit Sauerstoff und einer Fettsäure an einem festen Katalysator, der Palladium oder Platin und mindestens eines der Metalle Eisen, Nickel, Kupfer, Kobalt oder Chrom enthält.

Nach der DE-OS 22 17 452 kann man Diene, wie Butadien mit Sauerstoff und Carbonsäuren in Gegenwart von festen, Palladium und insbesondere Selen oder Tellur enthaltenden Katalysatoren zu ungesättigten Dioldiacetaten umsetzen. Nachteilig bei diesem Verfahren ist, daß die wirksamsten Katalysatorkombinationen die toxikologisch nicht unbedenklichen Elemente Selen und Tellur enthalten. Dies erfordert besondere Vorsichtsmaßnahmen bei der Herstellung der Katalysatoren. Außerdem sind die Katalysatoren in den sehr aggressiven Reaktionsgemischen etwas löslich. Spuren des Katalysators werden gefunden und können in die Folgeprodukte verschleppt werden. Außerdem bedeutet die Verschleppung von Katalysatorbestandteilen einen Verlust an Katalysator in den Reaktionsräumen.

Für diese Reaktion, die als Acetoxylierung von Diolefinen bezeichnet wird, sind auch schon Katalysatoren beschrieben, welche Palladium auf einem Träger in Kombination mit z.B. gelösten Kupfer-, Lithium- und anderen Salzen, wie Antimonchlorid als wirksame Komponente enthalten (vgl. Jap. Patentanmeldungen 77 12 170 und 77 18 162). Diese Katalysatoren besitzen jedoch mehrere technische Nachteile : Die Verwendung von teurem Essigsäureanhydrid ; ein oder mehrere Katalysatorbestandteile sind im Reaktionsgemisch gelöst, d.h. sie erfordern eine aufwendige Abtrennung und Rückführung ; niedrige Selektivität. Die zuletztgenannten Vorschläge sind daher schon gegenüber dem eingangs beschriebenen Verfahren nachteilig und nicht in technischem Maßstab ausführbar.

Nach den Angaben der DE-OS 20 12 903 werden für die Acetoxylierung von Butadien Trägerkatalysatoren auf Basis der Platinmetalle herangezogen, die als Promotoren Kupfer, Silber, Zink, Nickel, Chrom, Eisen, Kobalt, Cadmium, Zinn, Blei, Molybdän, Wolfram, Alkali- oder Erdalkalimetalle oder Carboxylate oder anorganische Salze dieser Metalle enthalten. Mit diesen Trägerkatalysatoren, die bei Temperaturen um 250 °C aktiviert werden, werden nur niedrige Butadienumsätze erzielt.

Es wurde nun gefunden, daß man Diester olefinisch ungesättigter 1,2- und/oder 1,4-Diole in hoher Ausbeute bzw. Selektivität und Raumzeitausbeute durch Umsetzung eines konkugierten Diolefins, wie Butadien, mit Sauerstoff und einer Fettsäure, wie Essigsäure, in der Flüssig- oder Gasphase an einem festen Katalysator, der Palladium und/oder Platin und wenigstens eines der Metalle Eisen, Nickel, Kupfer, Kobalt oder Chrom in reduzierter Form auf einem festen Träger enthält, herstellen kann, wenn man einen Katalysator verwendet, der durch Reduzieren der auf einem Träger aufgebrachten Metallverbindungen und Erhitzen auf über 400 °C erhalten wurde, wobei Platin bzw. Palladium und das weitere Metall oder die weiteren Metalle eine intermetallische Phase bilden.

Mischungen von z.B. Palladium und Platin sind nicht wirksamer als jedes Metall für sich.

Die Trägerkatalysatoren können in der für Palladium- und Platinträgerkatalysatoren üblichen Weise erhalten werden. Sie Können z.B. hergestellt werden, indem man einen Träger in einer Lösung dispergiert, die eine Palladium- oder Platinverbindung und eine oder mehrere Verbindungen der Übergangsmetalle enthält, das Lösungsmittel verdampft und den Rückstand in einem Gasstrom aus z.B. Wasserstoff oder Stickstoff, der mit einer reduzierenden Verbindung, wie Hydrazin, Methanol oder Formaldehyd beladen ist, reduziert. Die Reduktion des getrockneten Katalysators kann auch mit flüssigen Reduktionsmitteln geschehen.

Man kann den Katalysator auch herstellen, indem man Träger und Lösung gemeinsam mit einem z.B. alkalischen Fällungsmittel behandelt und die Ausfällung gewinnt und reduziert.

Eine sehr brauchbare Methode zur Herstellung der Katalysatoren ist die Fällung der Metalle aus wäßrigen Salzlösungen unmittelbar durch Reduktionsmittel, wie Formaldehyd, Hydrazin u.a. bei einem geeigneten pH-Wert (vgl. z.B. JACS *83* (1961) S. 4916).

Zweckmäßig werden die so erhaltenen rohen Katalysatoren noch in einem reduzierend wirkenden Gasstrom auf höhere Temperatur erhitzt.

Palladium bzw. Platin und die anderen Elemente können gleichzeitig oder in beliebiger Reihenfolge nacheinander auf dem Träger abgeschieden werden ; in manchen Fällen kann der Träger in Form einer löslichen Verbindung zugefügt und gemeinsam mit dem wirksamen Metall ausgefällt werden.

Mann kann jedes Reduktionsverfahren anwenden, durch welches die verwendeten Elemente in den metallischen Zustand überführt werden.

Es kommen Träger wie Aktivkohle, Bauxit, Bimsstein, Kieselgel, Kieselgur oder andere Formen der Kieselsäure, Magnesia, Ton, Tonerde in Frage. Die Träger können manchmal durch eine übliche Vorbehandlung, z.B. mit einer Säure, für ihre Zweckbestimmung verbessert werden.

Die zur Herstellung des Katalysators dienende Palladium-bzw. Platinverbindung ist nicht entscheidend ; z.B. können halogenierte Palladiumbzw. Platinverbindungen, wie Palladiumchlorid, die Platinchloride, ein Salz einer organischen Säure wie Palladium- oder Platinacetat, die Nitrate, Oxide verwendet werden. Man kann aber auch von anderen Palladium- oder Platinverbindungen, insbesondere komplexen Verbindungen, etwa Hexachloroplatinsäure, Natriumplatinsulfat, Ammoniumhexachloroplatinat ausgehen. Auch die erfindungsgemäß zu verwendenden Übergangsmetalle können weitgehend in Form frei wählbarer Verbindungen in die herzustellenden Katalysatoren eingeführt werden. Im allgemeinen wird man aus Zweckmäßigkeitsgründen zu löslichen Verbindungen greifen.

Gewöhnlich beträgt die Menge an katalytisch wirksamen Metallen auf dem Träger 0,1 bis 20 Gew.%, obgleich größere und kleinere Konzentrationen möglich sind. Die Entscheidung, welche Menge die günstigste ist, kann auch von wirtschaftlichen Überlegungen bestimmt werden. Im Zweifel kann sie durch orientierende Versuche gefunden werden.

Besonders bevorzugt und hervorragend geeignet sind Palladiumkatalysatoren der beschriebenen Art, die Aktivkohle als Träger und, neben 1 bis 10 % Palladium, bezogen auf das gesamte Katalysatorgewicht, etwa 1 bis 20 % Kupfer und/oder 1 bis 10 % Eisen enthalten. Höhere Konzentrationen an Palladium als die angegebenen können angewendet werden, haben aber im allgemeinen keinen besonderen Vorteil, da keine der Metallkonzentration entsprechende Steigerung der Raumzeitausbeute bzw. des Umsatzes mehr beobachtet wurde. Es ist erfindungswesentlich, daß diese Elemente nicht in Form zufälliger physikalischer Mischungen vorliegen, sondern mehr oder weniger hochgeordnete, sogenannte intermetallische Verbindungen bzw. Mischkristalle bilden. Die Bildung dieser intermetallischen Phasen ist durch Röntgenstrukturanalyse leicht feststellbar.

Es ist bekannt, daß intermetallische Phasen, an denen Platin oder Palladium beteiligt sind, eine kubisch-flächenzentrierte, kubisch-innenzentrierte oder auch tetragonale Struktur haben, wobei auch sogenannte Überstrukturen auftreten können. Diese Phasen sind an sich bekannt und z.B. in Gmelins Handbuch der Anorganischen Chemie, 8. Aufl., Bd. 68, Teil A, S. 617-652 (1951), beschrieben.

Zur Beschleunigung der Bildung intermetallischer Verbindungen bzw. der Mischkristalle kann es günstig sein, ternäre Metallsysteme zu verwenden. So hat sich beispielsweise gezeigt, daß die Bildung der $Pd_3Fe$-Phase durch Zusatz geringer Mengen von Chrom (z.B. 0,1 bis 1 Gew.%, berechnet auf Träger) stark beschleunigt wird. Hieraus ergibt sich, daß solche Katalysatoren unter Umständen bereits bei geringeren Temperaturen erhalten werden können als wenn nur zwei Metalle zu ihrer Bildung vorgesehen sind.

Eine wesentliche Maßnahme zur Erzielung von Katalysatoren, bei denen die in Rede stehenden Metalle intermetallische Verbindungen bzw. Phasen bilden, ist das Erhitzen. Im allgemeinen steht dabei die Dauer des Erhitzens und die Höhe der erreichten Temperatur in einem gewissen Zusammenhang. Es hat sich als zweckmäßig erwiesen, während 15 Minuten bis 4 Stunden auf über 400 bis 900 °C zu erhitzen, wobei im allgemeinen die kürzere Erhitzungszeit der höheren Temperatur zugeordnet ist. Da sich bei dem Erhitzen schließlich eine gewisse Stabilisierung des einmal erreichten katalytisch wirksamen Zustandes einstellt, ist die Anwendung einer bestimmten Höchstzeit im allgemeinen nicht erforderlich. Falls der Träger oder die Metalle unter den Bedingungen des Erhitzens zur Oxidation neigen, wird man zweckmäßig das Erhitzen in Gegenwart einer reduzierenden Atmosphäre, z.B. in reinem Wasserstoff vornehmen. Der Erfolg der erzielten Wärmebehandlung kann in jedem Falle sowohl durch die Bestimmung der katalytischen Aktivität als auch durch die Bestimmung der Röntgenstruktur erfolgen.

Ohne daß hierfür ein bestimmter Grund angegeben werden könnte, haben sich die erfindungsgemäß zu verwendenden Katalysatoren, die nachweislich intermetallische Phasen oder Gemische solcher Phasen enthalten, als katalytisch besonders aktiv erwiesen. Ganz besonders wirksam erwiesen sich Katalysatoren mit kubischflächenzentrierter Kristallstruktur, wie sie beispielsweise für die Verbindungen $Pd_3Fe$ bzw. $PdCu_3$ gefunden werden. Es hat sich jedoch gezeigt, daß es nicht erforderlich ist, die zu diesen Verbindungen führenden Ausgangsstoffe in stöchiometrischem Verhältnis einzusetzen. Wegen der Breite der entsprechenden Phasengebiete ist die Zusammensetzung in weiten Grenzen variierbar, wobei das Kristallgitter je nach Bedingungen Überstrukturen aufweisen oder auch verzerrt sein kann. Die Bestimmung der Gitterstruktur der intermetallischen Verbindungen kann in Anwesenheit der Trägerstoffe erfolgen. Neben der Ausbildung intermetallischer Phasen ist auch die Kristall(it) größe der wirksamen Verbindungen mitentscheidend für die katalytische Aktivität. Zur Erzielung einer hohen Aktivität sollte die Kristallitgröße so klein als möglich, z.B. kleiner als 100 nm, vorzugsweise kleiner als 50 nm, insbesondere kleiner als 20 nm sein. Die Kristallitgröße der Metallkomponente läßt sich durch geeignete Wahl der Herstellungsbedingungen variieren und röntgenographisch feststellen.

Die zu katalysierende Reaktion kann nach jedem bekannten Verfahren absatzweise oder fortlaufend durchgeführt werden, wie z.B. mit fixiertem Bett, Wirbelbett, Dreiphasenfließbett, wobei der jeweils gewählte Aggregatzustand des Reaktionsgemisches eine Rolle spielt.

Die Reaktionstemperatur liegt gewöhnlich zwischen 70 und 180 °C. In der Gasphase liegt sie vorzugsweise bei 120 bis 150 °C. In der Flüssigphase liegt die Temperatur z.B. zwischen 70 und 110 °C. Der Reaktionsdruck ist durch die Verfahrensweise gegeben und kann zwischen atmosphärischem Druck und z.B. 100 bar betragen.

Außer 1,3-Butadien sind auch andere konjugierte Diolefine, wie Isopren, 2,3-Dimethyl-1,3-butadien, 1,3-Pentadiene usw. der Reaktion zugänglich. Anstelle der reinen Diolefine können auch Kohlenwasserstoffgemische, welche neben den Diolefinen noch Monoolefine und Paraffinkohlenwasserstoffe enthalten können, verwendet werden. Als Fettsäuren im Sinne der Erfindung kommen Ameisensäure, Essigsäure oder Propionsäure in Betracht. Längerkettige Fettsäuren sind im allgemeinen weniger interessant, da für ihre Ester bisher keine spezielle Verwendung existiert, die nicht durch die Ester von z.B. Essigsäure ersetzt werden könnte.

Die nach dem Verfahren der Erfindung herstellbaren Butendiol-diester sind wertvolle Zwischenprodukte, z.B. für die Herstellung von Butendiol und Butandiol. Das in untergeordneten Mengen gebildete Butendiol-3,4-diacetat (Vinylglykoldiacetat) ist als Zwischenprodukt zur Herstellung von Vitaminen und anderen biologisch wirksamen Verbindungen geeignet.

Die bei der Acetoxilierung von Isopren erhaltenen 2-Methyl-1,4-diacetoxy-2-butene sind wertvolle Zwischenprodukte z.B. für die Synthese von Terpenverbindungen.

Wenn die Wirksamkeit des Katalysators nach einer gewissen Betriebszeit abfällt, kann sie vielfach durch geeignete Verfahren wiederhergestellt werden. So kann beispielsweise eine Belegung des Katalysators mit polymeren Verbindungen mit Hilfe geeigneter Lösungsmittel oder durch vorsichtige Behandlung mit sauerstoffhaltigen Gasen rückgängig gemacht werden. Wenn die Aktivität des Katalysators durch Oxidationsvorgänge beeinträchtigt worden ist, kann eine Regenerierung vielfach durch Behandlung mittels reduzierend wirkenden Verbindungen wie Hydrazin, Formaldehyd, Wasserstoff, Kohlenoxid, Methanol (dampf) usw. erreicht werden.

Beispiel 1

8,34 g PdCl$_2$ werden in einer Mischung aus 20 ml 4n-Salzsäure, 20 ml 30 %iger Salpetersäure, sowie einer Lösung von 8,96 g Kupferpulver in 84 ml 30 %iger Salpetersäure gelöst. 100 g Aktivkohle einer Korngröße von 0,4-0,8 mm, mit Salpetersäure gewaschen, werden vorgelegt, mit 100 ml Wasser angefeuchtet, die Lösung zugefügt und zum Sieden erhitzt. Unter Sieden wird eine Lösung von 70 g Hydrazinhydrochlorid, 400 ml Wasser und 300 ml wäßrige 15 %ige Ammoniaklösung nach und nach zugesetzt und 1,5 Stunden bei Siedetemperatur nachgerührt. Die Suspension wird filtriert, der Rückstand mit Wasser neutral gewaschen und mit 50 ml Methanol gespült. Man trocknet 10 Stunden unter vermindertem Druck bei 70 °C und behandelt im Röhrenofen 30 min. lang bei 800 °C mit Wasserstoff.

Die Strukturanalyse des Katalysators zeigt eine PdCu$_3$-Phase mit wenig PdCu. Die Gitterkonstante der PdCu$_3$-Phase beträgt 0,3696 nm, die mittlere Kristallitgröße 13 nm. Die gemessenen d-Werte in nm (CuK$_\alpha$-Strahler) sind in der nachfolgenden Tabelle angegeben.

Tabelle

| PdCu$_3$ | (hkl) | PdCu | (hkl) |
|---|---|---|---|
| 0.37 | 001 | 0.296 | 001 |
| 0.26 | 011 | 0.209 | 011 |
| 0.213 | 111 | 0.171 | 111 |
| 0.1848 | 002 | 0.148 | 002 |
| 0.1305 | 022 | 0.1325 | 021 |

543 g Essigsäure und 25 g Katalysator werden in einem 1-l-Vierhalskolben vorgelegt und unter N$_2$-Einleitung auf 85 °C erwärmt. Vier Stunden lang werden Butadien und Sauerstoff in einer Menge von je 3 l/h mit Hilfe eines Begasungsrührers (1 000 U/min) in die Flüssigkeit eingeleitet ; danach wird der Katalysator abfiltriert, das Filtrat durch Destillation vom überschüssigem Butadien und von nicht umgesetzter Essigsäure befreit und der flüssige Rückstand (69,9 g) analysiert. Er hat folgende Zusammensetzung :

74,7 Gew. % trans-1,4-Diacetoxy-2-buten
11,1 Gew. % cis-1,4-Diacetoxy-2-buten
10,2 Gew. % 3,4-Diacetoxy-1-buten
0,6 Gew. % trans-1-Hydroxy-4-acetoxy-2-buten
1,5 Gew. % cis-1-Hydroxy-4-acetoxy-2-buten
1,5 Gew. % 3,4-Dihydroxy-1-buten-monoacetat
0,3 Gew. % Butenylacetate
0,2 Gew. % Butadienylacetat

Die Ausbeute an Butendiol-Derivaten, d.h. Wertprodukten beträgt somit über 98,2 %.

Vergleichsversuch 1

Der Katalysator wurde in der Weise hergestellt, daß Palladium und Kupfer durch Reduktion mit Hydrazin auf der Kohle niedergeschlagen und nach Trocknung unmittelbar, d.h. ohne Erhitzen verwendet wurden. Die Ausbeute an Wertprodukt beträgt in diesem Falle weniger als 0,1 g. Das Röntgenbild des Katalysators zeigt verbreiterte Linien ohne erkennbare Struktur.

Beispiel 2

4,48 g Cu-Pulver (70,5 mg Äquivalente), gelöst in 42 cm$^3$ 33 %iger HNO$_3$, werden bei Raumtemperatur zu 5,31 g Pd(CH$_3$COO)$_2$ (23,7 mmol), gelöst in 75 cm$^3$ 50 %igem Äthanol, hinzugegeben.

50 g Aktivkohle, die zuvor mit Salpetersäure gewaschen wurde, werden vorgelegt, die vereinigte Metallsalzlösung wird zur Kohle hinzugegeben und das Gemisch am Rotationsverdampfer bei 85 °C und Wasserstrahlvakuum bis zur Trockene eingeengt.

Der Katalysator wird 2 Stunden bei 150 °C im Vakuumtrockenschrank, dann 2 Stunden bei 150 °C im Röhrenofen unter strömendem Stickstoff getrocknet. Anschließend wird 6 Stunden bei 200 °C, dann 6 Stunden bei 400 °C mit bei Raumtemperatur mit Methanol gesättigtem Stickstoff, schließlich 0,5 Stunden mit Wasser-

stoff (20 l/h) bei 800 °C aktiviert. Man läßt unter strömendem Stickstoff auf Raumtemperatur abkühlen.

An dem so hergestellten Katalysator, dessen Röntgenstrukturanalyse analog Beispiel 1 eine PdCu$_3$-Phase mit wenig PdCu zeigt, werden — wie in Beispiel 1 beschrieben — Butadien, Essigsäure und Sauerstoff zur Reaktion gebracht. Der Reaktionsaustrag (569.5 g) hat aufgrund der gaschromatographischen Analyse folgende Zusammensetzung:

9,32 Gew. % trans-1,4-Diacetoxy-2-buten
1,41 Gew. % cis-1,4-Diacetoxy-2-buten
1,32 Gew. % 3,4-Diacetoxy-1-buten
0,04 Gew. % trans-1-Hydroxy-4-acetoxy-2-buten
0,03 Gew. % cis-1-Hydroxy-4-acetoxy-2-buten
0,2 Gew. % 3,4-Dihydroxy-1-buten-mono-acetat
0,06 Gew. % Butenylacetate
0,06 Gew. % Butadienylacetat

Die Ausbeute an Butendiol-Derivaten, d.h. an Wertprodukten, beträgt 98,5 %.

Beispiel 3

8,3 g Palladiumchlorid (entspr. 5 g Pd) und 3,6 g Eisen-(II)-chlorid (FeCl$_2$.4H$_2$O) (entspr. 1 g Fe) werden bei 80 °C in jeweils 200 ml 6 n HCl gelöst, die vereinigten Lösungen zu 100 g Aktivkohle, wie in Beispiel 1 beschrieben, zugefügt und im Rotationsverdampfer bis zur Trocknung eingedampft. Der Rückstand wird unter vermindertem Druck 2 Stunden bei 150 °C getrocknet und anschließend im Röhrenofen bei 750 °C 30 Minuten im Wasserstoffstrom aktiviert.

Durch Röntgenstruktur wird eine Pd$_3$Fe-Phase mit einer Gitter-Konstante von 0,3845 nm ermittelt; die mittlere Kristallitgröße beträgt 20 nm.

543 g Essigsäure und der nach der vorstehenden Beschreibung erhaltene Katalysator werden in einem 1-l-Kolben unter Stikstoff auf 85 °C erwärmt und Butadiengas und Sauerstoff in einer Menge von je 3 l/h mit Hilfe eines Rührers mit hohler Welle eingeleitet; die Versuchsdauer beträgt 4 Stunden, dann wird wie in Beispiel 1 beschrieben aufgearbeitet; als Destillationsrückstand werden 30,8 g erhalten, mit folgender Zusammensetzung

66,1 Gew. % trans-1,4-Diacetoxy-2-buten
17,6 Gew. % cis-1,4-Diacetoxy-2-buten
13,0 Gew. % 3,4-Diacetoxy-1-buten
0,2 Gew. % trans-1-Hydroxy-4-acetoxy-2-buten
0,6 Gew. % cis-1-Hydroxy-4-acetoxy-2-buten
2,2 Gew. % 3,4-Dihydroxy-1-buten-mono-acetat
0,1 Gew. %Butenylacetate (Summe)
0,2 Gew. % Butadienylacetat

Die Ausbeute von Wertprodukten liegt hiernach bei 99,2 %.

Beispiel 4

Ein nach den Angaben des Beispiels 1 hergestellter Katalysator, der, bezogen auf sein Gesamtgewicht, 5 Gew. % Palladium und 1 Gew. % Nickel, enthält und der 15 Minuten bei 900 °C im Wasserstoffstrom aktiviert wurde, lieferte (unter den gleichen Bedingungen wie im Beispiel 1) 20,2 g Wertprodukte bei einer Selektivität von 95,1 %. Die Gitterkonstante der als katalytisch wirksam angesehenen Metallkomponente wurde zu 0,3811 nm bestimmt, die Kristallitgröße war 10,5 nm.

Vergleichsversuch 4

Der gleich zusammengesetzte, jedoch bei 400 °C im Methanol/Stickstoffstrom behandelte Katalysator erwies sich als fast unwirksam; es wurde nur 0,9 g Wertprodukt erhalten.

Beispiel 5

Ein Beispiel 3 entsprechender Versuch mit einem Pd/Co-Katalysator ergab 21,1 g Wertprodukt bei einer Selektivität von 93,3 % (Gitterkonstante der Metallphase: 0,3828 nm, mittlere Kristallitgröße: 14 nm). Ein Vergleichsversuch mit Aktivierung bei 400 °C im Methanol/N$_2$-Strom ergab nur 2,0 g Wertprodukt.

Beispiel 6

Ein mit Doppelmantel versehener aufrechtstehender Rohrreaktor (30 mm Durchmesser, Länge 680 mm) wird mit einem entsprechend Beispiel 1 hergestellten, aktivierten Pd/Cu-Katalysator befüllt und mittels Ölumlauf auf 90 °C aufgeheizt.

Am oberen Ende des Rohrs werden stündlich 200 ml Essigsäure, 3 Nl Butadiengas und 3 Nl Sauerstoff eingeleitet (Druck 1 bar).

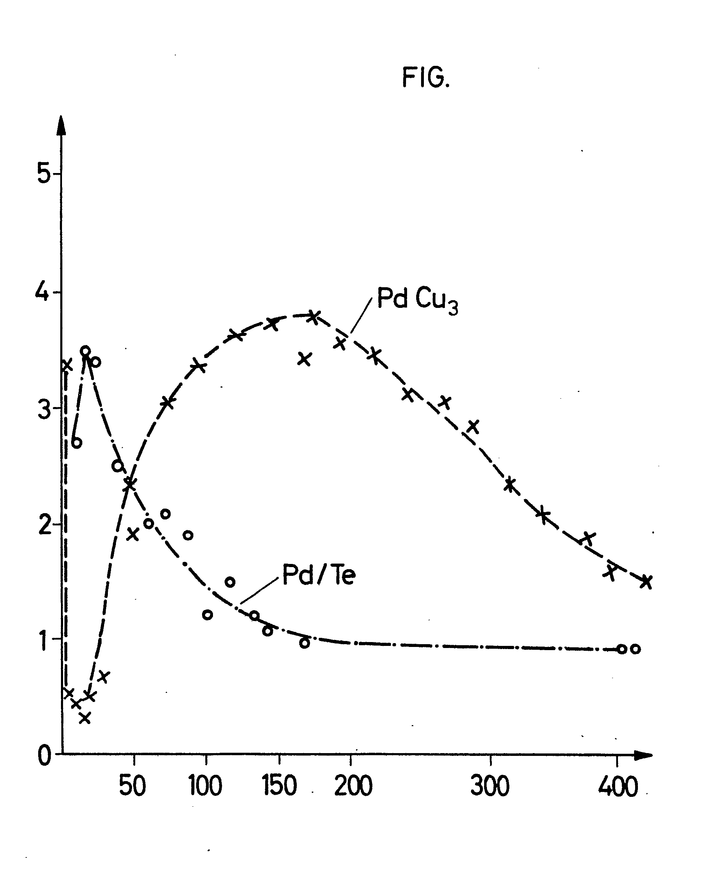

Das am unteren Ende anfallende flüssige Reaktionsprodukt wird gesammelt, auf Wertprodukte mittels quantitativer GC-Analyse untersucht und destillativ aufgearbeitet. Die zeitliche Abhängigkeit des Gehaltes des Austrages an Wertprodukten (= Summe der Butendioldiacetate) ist in der Figur wiedergegeben. In dem Diagramm ist die Summe der Wertprodukte in Gew. % über der Zeit in Stunden aufgetragen. Der Gehalt des Reaktionsgemisches an Butendiol-Verbindungen betrug — nach einer Anlauf-Phase — maximal 3,5 Gew. %. Der Palladiumgehalt im anfallenden Reaktionsgemisch beträgt 3 ppm, der Kupfergehalt 21 ppm.

Nach 400 Betriebsstunden hatte der Katalysator an Aktivität soweit nachgelassen, daß eine Regenerierung versucht wurde. Dies wurde durch Trocknen und Behandeln mit methanolbeladenem Stickstoff bei 200 °C und bei 400 °C, jeweils 4 Stunden erreicht. Nach dem Wiederingangsetzen des Versuchs hatte das Reaktionsgemisch einen Gehalt an Wertprodukten von 4,5 Gew. %.

Vergleichsbeispiel 6

Der in Beispiel 6 beschriebene Reaktor wird mit 250 ml eines Pd/Te-Katalysators, der nach der Vorschrift der DOS 22 17 452, Beispiel 13, hergestellt worden war, gefüllt und wie oben beschrieben betrieben. Die Konzentration an Wertprodukten gegen die Laufzeit ist ebenfalls in der Figur dargestellt.

Beispiel 7

In einem 1 Liter-Dreihalskolben mit Anschützaufsatz, Tropftrichter, Begasungsrührer, Innenthermometer, Gaseinleitungsrohr und Rückflußkühler mit aufgesetztem Trockeneiskühler werden nach dem Spülen der Apparatur mit Stickstoff 600 Teile Eisessig und 15 Teile eines entsprechend Beispiel 1 hergestellten Palladium und Kupfer enthaltenden Katalysators auf 95 °C erhitzt. Im Verlauf von 4 Stunden werden nach und nach 12 000 Volumenteile Sauerstoff eingeleitet und 36,5 Gewichsteile Isopren zugesetzt. Dann leitet man nach beendeter Zugabe bei 95 °C 30 Minuten lang Stickstoff durch das Reaktionsgemisch, läßt abkühlen und entfernt den Katalysator durch Filtrieren. Das Filtrat (592,8 Teile) enthält nach dem Ergebnis der GC-Analyse 5,8 Gew. % 2-Methyl-1,4-diacetoxy-2-buten (28 % cis, 72 % trans).

Beispiel 8

Pro Stunde werden 200 ml Essigsäure verdampft und zusammen mit je 10 l Sauerstoff und Butadien-1,3 durch ein Reaktionsrohr, das mit 100 g des Pd/Cu-Katalysators vom Beispiel 2 gefüllt und auf 140 °C aufgeheizt ist, geleitet. Die oberhalb vom Raumtemperatur siedenden Anteile der austretenden Dämpfe werden kondensiert und die Essigsäure vom Kondensat abdestilliert. Der verbleibende Rückstand enthält die isomeren Butendioldiacetate in der Massenverteilung (trans-1,2): (cis-1,2) : (-3,4) wie 7 : 1 : 2.

**Anspruch**

Verfahren zur Herstellung von Diestern von olefinisch ungesättigten 1,2- und/oder -1,4-Diolen durch Umsetzung eines konjugierten Diolefins, Sauerstoff und einer Fettsäure in der Gas- oder Flüssigphase an einem festen Katalysator, der Palladium und/oder Platin und wenigstens eines der Metalle Eisen, Nickel, Kupfer, Kobalt oder Chrom in reduzierter Form auf einem festen Träger enthält, *dadurch gekennzeichnet*, daß man einen Katalysator verwendet, der durch Reduzieren der auf einem Träger aufgebrachten Metallverbindungen und Erhitzen auf über 400 °C erhalten wurde, wobei Platin bzw. Palladium und das weitere Metall oder die weiteren Metalle eine intermetallische Verbindung bilden.

**Claim**

A process for the preparation of diesters of olefinically unsaturated 1,2- and/or 1,4-diols by reaction of a conjugated diolefin, oxygen and a fatty acid in the gas phase or liquid phase over a solid catalyst which contains palladium and/or platinum and at least one of the metals iron, nickel, copper, cobalt and chromium in a reduced form on a solid carrier, *characterized in that* a catalyst is used which has been obtained by the reduction of metal compounds applied to a carrier, and heating to above 400 °C, the platinum and/or palladium and the further metal or metals forming an intermetallic compound.

**Revendication**

Procédé de préparation de diesters de 1,2- et/ou 1,4-diols à insaturation oléfinique par réaction d'une dioléfine conjuguée, de l'oxygène et d'un acide gras en phase liquide ou gazeuse sur un catalyseur solide contenant du palladium et/ou du platine et au moins l'un des métaux fer, nickel, cuivre, cobalt ou chrome, à l'état réduit sur un support solide, caractérisé en ce que l'on utilise un catalyseur qui a été obtenu par réduction des composés métalliques appliqués sur un support et par chauffage à une température supérieure à 400 °C, le platine ou le palladium et le ou les autres métaux formant un composé intermétallique.

FIG.